# EUROPEAN PATENT APPLICATION

(11) **EP 1 001 030 A1**
(43) Date of publication of application: **17.05.2000**
(21) Application number: 98117900.5
(22) Date of filing: 22.09.1998
(51) Int. Cl.: C12N 15/86, A61K 35/76

(54) **Recombinant CELO virus and CELO virus DNA**

(71) Applicant: Boehringer Ingelheim International GmbH, 55216 Ingelheim (DE)
(72) Inventor: Cotten, Matthew, Dr., 1080 Wien (AT); Michou, Anne-Isabelle, Dr., 1030 Wien (AT)

(57) **Abstract**

Recombinant CELO virus or CELO virus DNA with a deletion at the right end of the viral genome that allows insertion of large pieces of foreign DNA. The virus is useful as a vaccine for animals, in particular birds, and for gene therapy and vaccine applications in humans.

## Description

The present invention relates to viral vectors and viral DNA.

Adenovirus has been studied for its role in human disease (25), as a model for many important discoveries in molecular biology, including mRNA splicing, DNA replication, transcription and cell transformation (reviewed in 44) and more recently as a powerful reagent for transient gene expression (12, 46). A detailed understanding of the adenoviral life cycle is well established (reviewed in 50). Since the initial efforts to use adenovirus as a gene transfer vector (18, 52, 28) the virus has gained in popularity as a vector and a number of methods of generating alterations in the viral genome to carry novel genes have been developed (2, 5, 11, 15, 21, 23, 26, 32, 38, 41, 43, 48 reviewed in 31, 49). Because of the ease of vector construction and purification, and because these vectors have a potent ability to transiently transduce novel genetic material into a variety of mammalian cell types in vivo, adenovirus vectors were used extensively in early efforts at clinical gene therapy.

Unfortunately, several features of the adenovirus type 5 (Ad5) based vectors initially used have limited the success in the initial applications. These included both the host immune response to adenovirus (reviewed in ref. 55) as well as the failure of the virus to efficiently enter certain target cell types (20, 58, 59). Thus, there is now an interest in adenovirus types that could provoke less aggressive host immune responses and could enter target cells with greater efficiency.

A large number of alternate adenovirus serotypes are known and may provide advantages in some applications over Ad5-based vectors. Additional adenoviruses that have recently been modified as vectors include the ovine adenovirus 287 (53, 56), the bovine adenovirus type 3 (40, 60), and the canine adenovirus (30). It is considered that these alternate serotypes would provide both a novel vector backbone to which there is no pre-existing immune response in the target host. Furthermore, because adenoviruses are extremely species specific in their replication capacity (50) a degree of security against inappropriate vector replication is gained by using an vector derived from a distant species of adenovirus.

It has been an object of the invention to provide an alternative adenovirus vector for use as a gene delivery vector.

To solve the problem underlying the present invention, the avian adenovirus CELO has been chosen to be modified. CELO (chicken embryo lethal orphan or fowl adenovirus type 1, reviewed in 39) was characterized as an infectious agent in 1957 (57). There are few serious health or economic consequences of CELO virus infection. CELO can be isolated from healthy chickens and in general, do not cause disease when experimentally re-introduced into chickens (10).

CELO virus is structurally similar to the mammalian adenoviruses (mastadenoviruses) with an icosahedral capsid of 70-80 nm made up of hexon and penton structures (33); the CELO virus genome is a linear, double-stranded DNA molecule with the DNA condensed within the virion by virus-encoded core proteins (33, 36). CELO virus has a larger genome than Ad5 (44 kb vs. ca. 36kb, ref. 6, WO 97/40180). The CELO virion has two fibers of different lengths at each vertex (24, 33, 35) rather than the single fiber of most other serotypes (reviewed in 50). The CELO virus is not able to complement the E1A functions of Ad5 and CELO virus replication is not facilitated by Ad5 E1 activity (37). The complete DNA sequence of CELO (6, WO 97/40180) revealed additional differences between CELO virus and the mastadenoviruses including the absence of sequences corresponding to the Ad5 early regions E1A, E1B, E3 and E4. The CELO genome contains approximately 5 kb of sequence at the left end and 12 kb at the right end, rich in open reading frames, which have no sequence homology to Ad5 but probably encode the early functions of the virus.

When developing CELO into a gene delivery vector, it has been considered that the virus is naturally defective in mammalian cells and this property should limit the possibility of complementation by wildtype mammalian adenovirus. The CELO virion has increased DNA packaging capacity and much greater physical stability than the virion of Ad5. One practical feature of CELO is the ability to grow the virus in chicken embryos, a system of low cost and high convenience (9, 33).

In the experiments of the present invention, the frontier sequences of CELO, i.e. the leftmost 5 kb and rightmost 13 kb of the CELO genome that are largely unexplored and are not common to other adenoviruses, have been examined. Viral genetics have been used to characterize the requirements for replication of these left and right end CELO sequences. Viral sequences have been deleted in discrete steps in order to identify the regions essential or non essential for replication. To facilitate monitoring the replication of the mutants, a luciferase expression cassette was inserted in place of the deleted sequences. The modified CELO genomes were engineered as bacterial plasmids using homologous recombination in E. coli (5). Subsequently, after their release from the plasmid backbone by enzymatic digestion, the viral genomes were transfected into a chicken cell line supporting wildtype CELO replication. Transfection of the large viral DNA molecules (about 50 kb) was facilitated by optimizing the polyethyleneimine (PEI) mediated transfection method (1, 3). Furthermore, with all mutations, a second transfection was performed with a plasmid bearing the CELO sequences that were deleted from the mutant to determine if the mutation could be complemented. Monitoring the production of luciferase in cells treated with lysates of the initial transfectants allowed us to determine if virus replication and production of transducing viral particles had occurred. These strategies were used to determine the essential portions of both left and right frontier sequence. As anticipated, some of the sequences were required in cis and presumably these contain packaging signals, transcriptional promoters or other transcription signals.

The present invention relates to recombinant CELO virus and CELO virus DNA that have the region spanning nucleotides 41731-43684 of the CELO wild type virus genome deleted.

This CELO virus (DNA) and its derivatives have been designated CELO AIM46 or CELO AIM46 derivatives, respectively.

In an embodiment, the invention is directed to a CELO AIM46 derivative with a deletion that spans the region nt 41523-43684.

In a further embodiment, the invention is directed to a CELO AIM46 derivative with a deletion that spans the region nt 41002-43684.

In a further embodiment, the invention is directed to a CELO AIM46 derivative with a deletion that spans the region nt 40065-43684.
The modified viruses of the invention, which are derived from CELO virus, contain deletions at the right end of the genome, are replication competent and can carry at least 1.7 kb more DNA than the wildtype genome.

More specifically, the CELO virus and CELO virus DNA of the invention carry a deletion of wild type CELO sequences for insertion of an expression cassette for foreign genes in the CELO virus genome, the deletion spanning approximately the region from nt 40,000 to approximately within 200 bp of the right terminus of the virus genome. The region that may be deleted is thus defined by the last three rightward open reading frames encoding peptides of greater than 99 amino acid residues, the terminal repeat function of the virus normally residing within the last 100 - 200 bp of the virus genome remaining undisrupted. Optionally, the CELO virus and CELO virus DNA of the invention may, in addition, have a deletion in the region defined by the open reading for the CELO duTPase (794-1330).

The CELO nucleotide sequence numbering used in the present invention is derived from reference 6, WO 97/40180 and GenBank U46933, which describe the sequence of the wild type CELO virus genome.

Since CELO AIM46 and its derivatives defined above allow the insertion of large pieces of foreign DNA, they are useful as starting material for producing CELO virus vectors.

It has surprisingly been found that a 3.3 kb insert is tolerated by CELO AIM46 without impairing virus growth, thus it may be expected that larger DNA up to approximately 10 kb inserts may be acceptable for the virus. The maximum size limit can be easily tested by the artisan in routine experiments by gradually increasing the insert size and determining the viral growth parameters, as in the experiments of the present invention.

Apart from allowing the insertion of an expression cassette for genes, the recombinant CELO virus of the invention has been shown to be able to replicate without complementation both in LMH cells and in chicken embryos.

A further advantage of the recombinant CELO virus of the invention is that it yields quantities of virus that are comparable to wild type CELO. Furthermore, it was found that AIM46 vectors transduce mammalian cells comparable to Ad5 vectors, demonstrating efficient mammalian cell receptor binding and/or entry activity by CELO vectors.

Since the CELO AIM46 deletion of 41731 - 43684 removes only part of the open reading frame beginning at nt 41002, the entire ORF should be dispensable and may therefore be deleted. Thus, the deletion may encompass the sequence from nt 41002-43684.

In an embodiment of the invention, the gene of interest is inserted in other genomic sites than the deleted region from nt 41731-43684, 41523-43684, 41002-43684 or 40065-43684, respectively; in this case the vector provides additional space for insertion of further foreign genetic material. Any region that proves to be dispensible may be chosen, a preferred region for inserting foreign DNA is at the dUTPase open reading frame (nt 794-1330).

The virus of the invention grows to wildtype levels and forms the basis of a replication competent vaccine strain. CELO AIM46 was also used to begin analysis of the cell tropism of CELO in regard to both species and cell types, generating information that is important for developing applications of this vector. CELO AIM46 and its derivatives comprising additional modifications, can be used to deliver genes into avian cells with efficiencies of 10-100 fold better than an Ad5 vector bearing the same marker gene. Surprisingly, in a variety of mammalian cell types, CELO AIM46 functions with efficiencies that are comparable to an Ad5 vector demonstrating the utility of CELO vectors for mammalian gene transfer applications.

In a further embodiment, the invention relates to the production of recombinant CELO AIM46 virus and its derivatives.

For production of recombinant CELO virus, the CELO virus DNA is introduced into cells that support CELO virus replication. Any standard method for gene transfer may be used for introducing the DNA, e.g. transfection, microinjection, etc. Preferably, the viral DNA is introduced into the cells by polyethylenimine-mediated transfection, as described by (3).

The cells that support CELO virus replication and are thus useful for CELO virus production, may be selected from immortalized cells like LMH (27) or from primary avian embryonic cells, in particular kidney or liver cells. To identify useful cell lines, cells are tested for infectability and the ability to amplify an inoculum of virus.

Alternatively, the recombinant CELO-Virus may be produced by introducing CELO virus into chicken embryos, preferably by first transfecting the cells described above with CELO virus DNA and keeping the cells in culture for a period of time to produce a quantity of virus sufficient for injection into, and amplification of, the virus in chicken embryos.

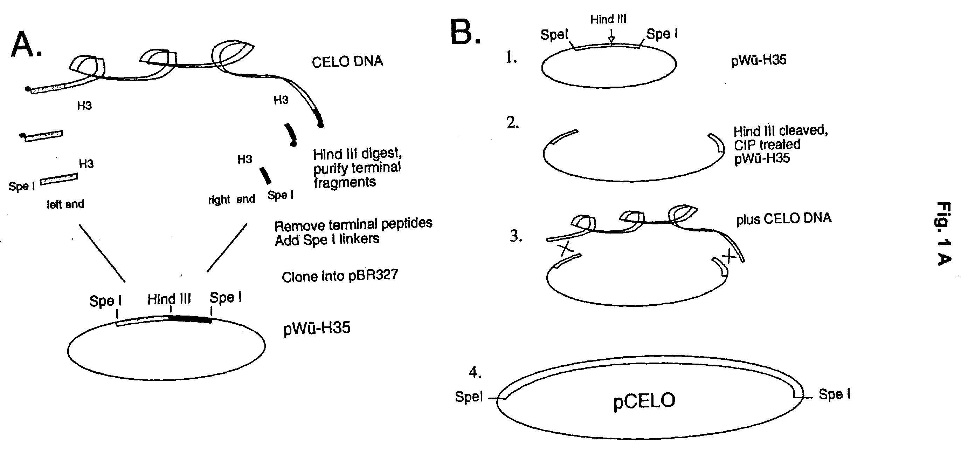

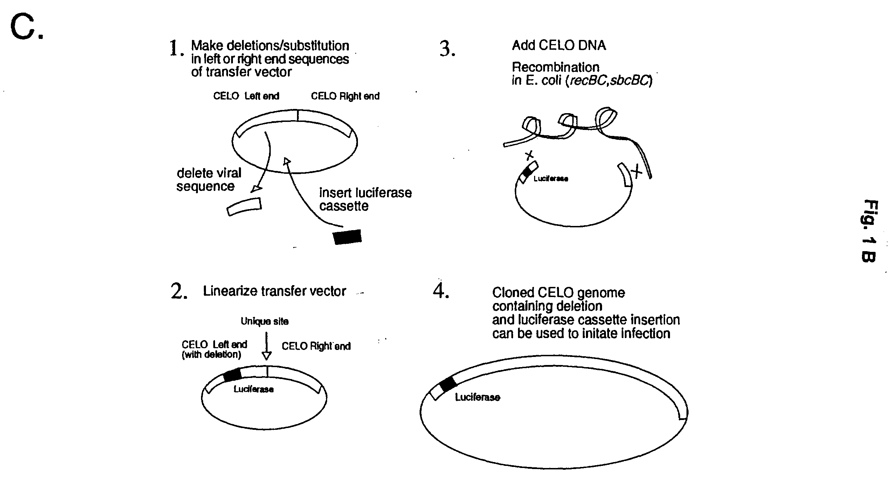

In order to produce recombinant CELO virus, regions that are dispensable for viral replication are first identified. To this end, subfragments of the CELO genome which are large enough to facilitate reconstruction into the CELO genome and small enough to possess a large number of unique restriction sites, are cloned on a plasmid. The plasmid is manipulated with standard restriction enzyme digestion to delete various fragments of the CELO genome and to insert a reporter gene construct in place thereof. This manipulated fragment is then inserted into the full CELO genome, e.g. in a bacterial plasmid, replacing the corresponding wild type fragment using standard ligation or recombination methods. The modified CELO genome is then released from the plasmid backbone by restriction digest and introduced into cells that support CELO virus replication, as exemplified in Figure 1.

In the case of CELO AIM46 the subfragment contains CELO sequences from nt 1-5503 and 30502-30644 and 40064-43804. An EcoRV fragment from 41731-43684 is deleted and replaced with an expression cassette, e.g. a luciferase expression cassette, to generate pAIM44. This is linearized with HpaI, recombined with wild type CELO DNA to generate pAIM46. CELO sequences are excised from the plasmid by SpeI digestion and introduced into LMH cells to initiate virus replication.

Alternatively, recombination can be performed in avian cells that support viral replication, e.g. LMH cells, by introducing a modified CELO subfragment that contains a deletion/insertion with a second CELO fragment such that overlapping homology between the two fragments allows recombination to full length CELO genome bearing the desired deletion/insertion.

In the experiments of the present invention, the CELO genome regions that are essential for virus replication were identified. Most importantly, a region in the left end of the virus genome that can be deleted and supplied in trans has been identified. This region may therefore be useful for establishing a complementing cell line. Furthermore, a region in the right end of the genome has been identified that can be deleted with no detectable effects on virus replication in either cell culture or in embryos. It has been shown that an expression cassette for foreign genes can be inserted in this region. It has been demonstrated that CELO vectors can package an additional 1.7 kb of DNA sequence over the wildtype genome size, which is already 8 kb larger than Ad5 vectors. Replication competent OSLO vectors bearing either a luciferase expression unit or an EGFP expression unit were developed. These vectors were monitored for their ability to transduce a variety of avian and mammalian cell types. As expected, the CELO vectors work much better than an Ad5 vector in avian cells. However, in all mammalian cell types tested, the CELO vectors surprisingly functioned with transduction efficiencies comparable to Ad5 vectors.

The vectors of the invention have vaccine applications in avian species where viral replication can promote immune responses. The ability to propagate the virus in inexpensive chicken embryos facilitates production of large quantities of the vector for any of these applications.

For vaccine applications, the foreign DNA encodes one or more antigens eliciting an immune response in the individual.

A variety of non-human adenovirus vectors have been developed in recent times including a vector based on the bovine adenovirus type 3 (40), on the ovine adenovirus OAV287 (29, 53) and on the canine adenovirus type 2 (30). The are several justifications for pursuing these alternate viral subtypes. For vaccine applications in their non-human hosts, these viruses, if properly modified, may provoke more effective immune responses than a human adenovirus based vector. Furthermore, more robust immune response might be expected from a replication competent virus; thus a vector is most useful in a host where replication is partially or fully permissive. This is not the case with human adenovirus based vectors in nearly all nonhuman hosts. In this regard, the CELO vectors of the present invention, CELO AIM46 and its derivatives, are ideally suited for avian vaccine applications.

Examples for avian vaccine applications are given in WO 97/40180, which is fully incorporated by reference herewith. Further examples for antigens that may be carried by the virus for vaccination applications are antigens of Chicken coccidia, e.g. Eimeria acervulina, Eimeria brunetti, Eimeria maxima, Eimeria mitis, Eimeria necatrix, Eimeria praecox and Eimeria tenella (61, 62, 63), and antigens of infectious bursal disease virus (IBDV; 64).

The recombinant CELO virus vectors of the invention are also useful in human vaccine applications, in this case, the foreign DNA encodes an antigen derived from a human pathogen.

CELO vectors based on the CELO genome modified according to the present invention are also useful for gene transfer applications in mammalian systems; an additional argument for pursuing a non-human adenovirus coming from the experience with human adenovirus in human gene therapy applications. Pre-existing immune responses to human adenovirus can impair the initial transduction by human adenovirus based vectors or might exacerbate the cellular immune response to transduced cells. A patient may have no immune experience with an adenovirus from a distant species (although 2 of 7 patients had neutralizing antibodies to the canine adenovirus vector; 30) and initial transductions will not be compromised by the host response to viral antigens. Except for certain agricultural workers, a previous immune exposure to CELO antigens would not be expected in most of the human population. CELO vectors might therefore have an advantage over vectors based on more common human adenovirus serotypes.

An additional conceptual advantage of CELO based vectors of the invention is that CELO, like the bovine, ovine, and canine adenoviruses, is naturally replication defective in human cells. Thus, replication of these vectors is not going to occur in human patients even in the presence of a wildtype human adenovirus infection.

Furthermore, the ability to generate a replication defective CELO vector will be facilitated by the deletion analysis performed in the present invention. The findings of the present invention provide the basis for constructing cell lines expressing CELO complementing functions.

For gene therapy applications, the foreign DNA may comprise any one or more DNA molecules encoding a therapeutically active protein. Examples are immunomodulatory proteins like cytokines; receptors, enzymes, proteins effecting apoptosis, etc. For tumor vaccine applications, the foreign DNA encodes one or more tumor antigens or fragments thereof, preferably in combination with a cytokine.

Examples for human vaccine applications, gene therapy and tumor vaccine applications are given in WO 97/40180, which is fully incorporated by reference herewith.

The CELO virus of the invention is also useful for producing any protein of interest. In this embodiment of the invention, the CELO virus is engineered, as described above, by introducing the cDNA encoding the protein of interest into one of the insertion sites of the recombinant CELO DNA of the invention. Virus may be obtained by replication in suitable cells, as described above, and the recombinant virus is introduced, preferably by injection into the allantoic cavity of an avian embryo. Preferably, approximately 10⁹ particles are introduced into the allantoic cavity of 7 to 9 day old chicken embryos, which are subsequently incubated for three to four days at 37°C.The recombinant material is then recovered from the allantoic fluid.

### Brief description of the figures:

- Figure 1.: Construction of plasmids carrying wildtype and mutant CELO genomes
- Figure 1A.: Construction of a plasmid bearing the termini of the CELO genome
- Figure 1B.: Cloning of full length CELO genome as a bacterial plasmid
- Figure 1C.: Cloning of modified versions of the CELO genome
- Figure 2.: Analysis of CELO left end mutations Figure 2, upper panel: Open reading frames Figure 2, lower panel: Analysis of replication
- Figure 3.: Analysis of CELO right end mutations Figure 3, upper panel: Open reading frames Figure 3, lower panel: Analysis of replication
- Figure 4.: PCR analysis of wt CELO vs. CELO AIM46
- Figure 5.: Immunofluorescence, monitoring the replication of wildtype CELO vs. CELO AIM46
- Figure 6.: Heat stability of AIM46 vs. AdLuc
- Figure 7.: Tropism of CELO vs. Ad5
- Figure 8.: Transduction of EGFP using recombinant Ad5 or CELO vectors
- Figure 9.: Analysis of CELO right end mutations Figure 9, upper panel: Open reading frames Figure 9, lower panel: Analysis of replication

Unless otherwise stated, the following materials and methods were used in the Examples:

### a) Cloning the terminal fragments of the CELO genome

The two terminal HindIII fragments of CELO were cloned. CELO genomic DNA (CsCl purified) was digested with HindIII, the 1601 bp left end fragment and the 959 bp right end fragment were purified from a low melting agarose gel. The 5' ends of adenovirus genomes are linked by a phosphodiester bond to a serine residue on the viral terminal protein (22, 47). The peptide remaining after proteinase digestion must be removed to allow ligation and cloning. Accordingly, the terminal peptides were removed from the DNA fragments by adding NaOH to 0.3 N and heating at 37 °C for 90 minutes (22). The solutions were then cooled to room temperature, Tris pH 7.4 was added to 0.1 M and HCl was added to 0.3 M to neutralize the NaOH. The fragments were heated to 56°C for 20 minutes and slowly cooled to room temperature (1 hour) to facilitate reannealing. The DNA was then purified (Qiaquick column, Qiagen), SpeI linkers (New England Biolabs cat # 1085) were added and each fragment was cloned via SpeI and HindIII sites into a pBR327 (GenBank L08856, ref. 51) derivative containing an SpeI site in a destroyed EcoR1 site (see figure 1A). Both the left and the right terminal HindIII fragment were cloned in this manner and DNA sequence analysis was performed to verify both terminal 300 bp of both fragments.

Subsequently, the two CELO end fragments were cloned into a pBR327 derivative containing an SpeI site, a destroyed EcoRI site and a ClaI/BamHI excision to remove the second Hind III site, creating the plasmid pWü-H35 (see figure 1A).

### b) Cloning the entire CELO genome

HindIII-linearized, alkaline phosphatase treated pWü-H35 vector was mixed with purified CELO virus DNA and introduced into electrocompetent *E. coli* JC8679 (17, 42) by electroporation. Recombination between the CELO terminal sequences on pWü-H35 and the termini of the CELO genomic DNA generated an plasmid containing a full length CELO genome (pCELO) flanked by SpeI sites (figure 1B).

### c) Modifications in the left end of the CELO genome

The luciferase cassette containing the Cytomegalovirus immediate early enhancer/promoter, the luciferase cDNA (14) followed by a rabbit βglobin intron/polyadenylation signal was derived from pCLuc (45), modified by PCR to add flanking BamH1 sites and cloned into pBlueScript II (SK) to generate pBlueLuc. For most of the CELO insertions, the luciferase cassette was isolated from pBlueLuc by BamH1 digestion and the termini were made blunt by treatment with Klenow enzyme.

Modification of the left end CELO region were made using pAIM3, which contains, on a pBR327 backbone, the CELO left end (nt 1 to 5501) and a portion of the right end (nt 30500 to 30639 and nt 40065 to 43804; derived by removing an Asp718 fragment from pWüHpa). The deletions in the CELO genome involved digestion of pAIM3 at two enzyme sites in the CELO left end sequence, excision of the sub-region followed by insertion of a luciferase cassette. All manipulations were confirmed by restriction analysis and sequence analysis. This strategy was used to generate the transfer plasmids pAIM7, 16, 22, 23 and 24 (see Table 1). The CELO nucleotide sequence numbering is derived from reference 6 and GenBank U46933.

### d) Generation of recombinant CELO genomes

The plasmids pAIM7, 16, 22, 23 and 24 were linearized by double digestion using Asp718 and HpaI, and recombined with purified CELO DNA using homologous recombination in *E coli* BJ5183 (5, 13) to generate the CELO genome plasmids pAIM11, 21, 25, 26 and 27. Figure 1C and Table 1 illustrate the applied technique:

Figure 1C shows the cloning of modified versions of the CELO genome. Step 1: Transfer vectors were produced by manipulating subfragments of the CELO genome, as either pWü-H35 (with the terminal HindIII fragments of CELO) or pWüHpa (with the terminal Hpa I fragments of CELO) using standard ligation cloning methods in order to delete portions of the CELO genome and insert a luciferase cassette. Step 2: the linearized transfer vector was recombined with wildtype genomic CELO DNA. Recombination occurs in two ways, either to include the deletion/luciferase cassette or to exclude the deletion/luciferase cassette to generate a wildtype CELO plasmid. Plasmids bearing the desired mutation were identified by restriction enzyme digestions and sequencing and used to initiate virus infection. (all constructs were sequenced across the deleted regions to verify the construct; see Table 1).

### e) Modifications in the right end of the CELO genome

Using methods similar to those described above, plasmids containing both the left and right HpaI fragments of CELO were generated and manipulated to insert the luciferase cassette and to remove an EcoRV fragment from either 33358-43684 (pAIM43) or 41731-43684 (pAIM44). These plasmids were linearized at the unique Hpa I site and recombined in BJ5183 cells with wildtype CELO DNA to generate either pAIM45 or pAIM46.

### f) Evaluation of the recombinant CELO genomes on LMH cells and preparation of viral stocks.

The recombinant CELO plasmids were digested with SpeI to release the viral genome from the plasmid, extracted with phenol, with chloroform and then purified by gel filtration (Pharmacia Nick Column) equilibrated with TE.

Transfection complexes were prepared using a modification of the PEI technique (1, 3). The DNA was condensed with PEI in two steps as follows: PEI MW 2000 (2.5 µl of 10 mM PEI in 125 µl HBS (150 mM NaCl, 20 mM HEPES, pH 7.4)) was added dropwise to 3 µg of DNA diluted in 125 µl of HBS. The sample was incubated at room temperature for 20 minutes. Subsequently, PEI MW 25000 (3.5 µl of 10 mM in 125 µl of HBS) was added dropwise to the sample and the complex incubated at room temperature for an additional 20 minutes.

Leghorn Male Hepatoma (LMH) cells (27) were seeded the day before transfection in 24 wells plates at 7x 10⁴ cells/well (24 well dish). For transfection, the cell culture medium was replaced by 400 µl DMEM supplemented with 10 µg/ml polymyxin B (no serum). The transfection complex (90 µl per well) was added to the cells for 4 hours at 37°C after which the medium was replaced with fresh, serum containing medium. Transfection effiency was monitored by measuring luciferase activity in cell lysates at 24 hours post-transfection.

To test for amplification of virus, cleared lysates from transfected or transduced cells were prepared as follows. Cells plus supernatant were harvested, collected by centrifugation and the cell pellets were resuspended in 2 ml of processed supernatant. The material was frozen and thawed 3 times, sonicated in a bath sonicator to release viral particles, the cell debris was removed by centrifugation, and the cleared lysate used for further amplification on fresh cultures of LMH cells. CELO purification by CsCl gradient was performed as previously described (9). Virus was quantified based on protein content with the conversion factor of 1 mg/ml protein equal to 3.4 x 10¹² virus particles/ml (34).

### g) Construction of EGFP expressing CELO AIM53

The luciferase cDNA in pAIM46 was replaced by an EGFP cDNA to generate pAIM53. The replacement was obtained by homologous recombination in E *coli* between pAIM46 linearized at the unique PacI site in the luciferase cDNA, and pAIM52, a transfer plasmid carrying an EGFP cDNA under the control of the same CMV promoter and β-globin intron and polyadenylation signal used in the luciferase cassette of pAIM46, thus providing homologies for recombination.

### h) Generation of recombinant type 5 adenoviruses

AdLuc: The luciferase cassette was cloned via the flanking BamHI sites into pDE1sp1B (2), to produce pDE1sp1BLuc, with the luciferase cDNA in the same orientation as E1 transcription. Recombinant virus was generated using recombination after cotransfecting pDE1sp1BLuc with pJM17 (2) into 293 cells (19). 10 days post transfection, cells lysates were prepared, used to infect fresh 293 monolayer and virus was amplified from a single plaque. The virus stock used here was prepared from material that was subsequently passed through 2 additional rounds of plaque purification, amplified, purifed by banding in CsCl and quantified by protein content (1 mg/ml protein = 3.4 x 10¹² virus particles/ml; ref. 34).

AdGFP: A fragment containing the CMV promoter, EGFP coding region and SV40 poly A sequences was excised from the pEGFP-C1 (Clontech) using Ase1/Mlu1. Overhanging ends were filled in by Klenow and cloned into the EcoRV site of pDE1sp1B (2) with the EGFP cassette in the same orientation as E1 transcription. Recombinant virus was generated as described above using recombination with pJM17 in 293 cells.

### i) Analysis of heat stability of viruses

CELO AIM46 and AdLuc were diluted to 4 x 10⁹ particles/100 µl concentration in HBS (final glycerol concentration was 2.4 % (vol/vol.) and exposed for 30 minutes to temperatures ranging from 48 to 68°C. Subsequently, aliquots of the virus were tested for the ability to transduce luciferase activity into either A549 or CEF38 cells.

### j) Immunofluorescence

LMH cells were plated on gelatin-coated glass slides (Labtek, Nunc) at 10⁵ cells/chamber and infected the next day with CELO AIM46 or wilde type CELO virus at 500 viral particles/cell in DMEM medium containing 2% FCS. At the indicated time points after the infection, cells were fixed in cold methanol:acetone (1:1) at room temperature and CELO proteins were visualized by immunofluorescence as follows: nonspecific binding sites were blocked using PBS+1% BSA at room temperature for 1 hr. Polyclonal anti-CELO antibody was diluted 1:1000 in PBS+1% BSA and incubated for 1 hr. After three, 5 minute washes in PBS at room temperature, a goat-anti-rabbit (Boehringer-Mannheim) detection antibody coupled to FITC (1:400 dilution) was added in PBS+1% BSA. The slides were again washed, DAPI was included in the last wash for visualization of the nuclei and the slides mounted in MOWIOL for examination by fluorescence microscopy.

### k) Generation of anti-CELO virion polyclonal serum

Rabbits were injected with 100 µg of CsCl purified, heat inactivated (70 °C, 60 minutes) CELO virions in complete Freund's adjuvant, boosted at 2, 4 and 5 weeks with 100 µg of CELO in incomplete Freund's adjuvant and serum was collected subsequently. Western analysis demonstrated that the pooled sera used here reacted specifically with all major CELO capsid proteins but not with lysates of non-infected avian cells.

### l) Additional reagents

Wildtype CELO (FAV-1, Phelps) (Avian adenovirus Type 1, Chicken Embryo Lethal Orphan; CELO American Type Culture Collection: ATCC VR-432; Strain: Phelps; (57)) was purified from infected chicken embryos as previously described (9).

The LMH cell line (27) was obtained from ATCC No. CRL-2117, the A549 cell line was also obtained from the ATCC No. CCL-185, and normal human dermal fibroblasts were obtained from Clonetics and were used between passage 5 and 15. The chicken fibroblast cell line designated CEF38 was established from fibroblasts according to known methods; it allows CELO virus entry but does not support CELO replication. All four cell types were cultured in DMEM/10% FCS. The 293 cell line (19) was obtained from the ATCC No. CRL-1573 and was cultured in MEMalpha with 10% newborn calf serum.

### Example 1

### Construction of a plasmid copy of the CELO genome

Initially the terminal HindIII fragments of CELO were purified from CELO viral DNA, treated with base to remove the terminal peptides, linkers encoding SpeI restriction sites were added and the two terminal fragments were cloned in the correct orientation into a low copy number plasmid (Figure 1A depicts the construction of a plasmid bearing the termini of the CELO genome. CELO genomic DNA was digested with HindIII, the two terminal fragments were isolated, treated to remove the terminal peptides and cloned as SpeI, HindIII fragments after addition of SpeI linkers generating the plasmid pWü-H35.). This plasmid encoding the two ends of the virus (pWüH-35) was linearized with the unique HindIII site and recombined with CELO genomic DNA to generate a full length CELO genome flanked by SpeI sites on a bacterial plasmid (see Figure 1B, which shows the cloning of full length CELO genome as a bacterial plasmid. pWü-H35 was linearized with HindIII and recombined with CELO genomic DNA to generate the full length plasmid clones of the CELO genome. The natural terminal repeats were flanked by SpeI sites to allow excision of the viral genome from the bacterial plasmid. There are no SpeI sites within the CELO genome.). Several independent clones of the viral genome were obtained and the correct structure was verified by restriction analysis and by the production of virus upon transfection.

### Example 2

### a) Analysis of unique sequences required for virus replication.

A screening method for determining the requirement of CELO sequences for virus replication was developed. Deletions were first introduced into bacterial plasmid copies of the viral genome using homologous recombination in bacteria. In all cases the deleted viral sequences were replaced with a luciferase cassette to allow monitoring of both the initial transfection efficiency into cells that support wildtype virus replication and the replication and transduction potential of the mutant virus in subsequent passages. As will be shown below, the CELO genome allows the insertion of at least 1.7 kb of sequence beyond the wildtype genome size, thus the concern that introducing the luciferase cassette itself might impair replication was not realized. The mutant viral genomes were excised from the plasmid and transfected into LMH cells either alone, to determine if the deletion removed essential DNA sequences or with a plasmid bearing the region of the CELO genome that spans the deletion, to determine if complementation of the deletion could occur. Five days after transfection, the cells were lysed, a portion of the lysate was assayed for luciferase activity to monitor transfection efficiency and a second portion was used to infect a fresh monolayer of LMH cells. After another 5 day period, the cells were monitored for cytopathic effect, lysates were prepared and assayed for luciferase and a portion was again used to infect fresh LMH cells.

### b) Analysis of CELO left end

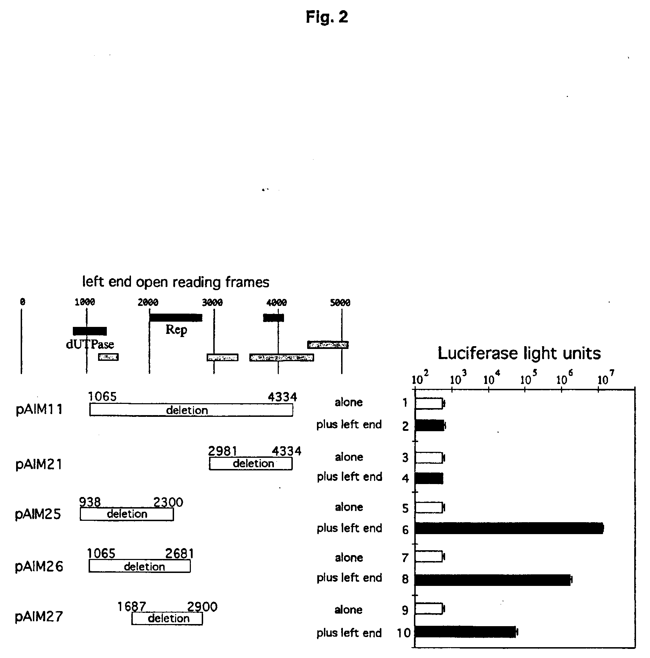

Using the strategy described in a), the unique left end CELO sequences were analyzed for replication function. The map of the left end open reading frames of 99 amino acids and larger is shown in Figure 2A: Open reading frames of greater than 99 amino acid residue in the left ca. 5000 nt of the CELO genome are indicated in either black (rightward transcription) or grey (leftward transcription). The open reading frames coding for a deoxyUTPase (dUTPase) and a protein with parvovirus REP homologies (REP) are indicated. An open reading frame encoding a functional dUTPase is found at position 784 (54). An open reading frame beginning at position 1991 encodes a protein with significant homology to the parvovirus REP gene. An additional five open reading frames are also indicated.

Figure 2 shows the analysis of replication. The nucleotide numbers of deletions introduced into the CELO genome are listed in the upper panel of figure 2 and in table 2. The modified CELO genomes were linearized with SpeI to release the genome from the bacterial plasmid and transfected into LMH cells either alone, or in the presence of a plasmid (pB5.5) bearing wildtype CELO sequences from 1-5501 ("plus left end"). At 5 days post transfection, cells were harvested, lysed by freeze/thawing and sonication and the lysates were applied to a fresh LMH culture. This amplification was repeated twice and equal aliquots from the third passage of virus were tested for their ability to transduce luciferase activity in LMH cells. The average of three transductions with the standard deviations are indicated.

Mutant genomes were constructed that removed first the entire region (pAIM11) or deleted single or small groups of open reading frames (pAIM21, 25, 26, 27). When introduced into LMH cells by transfection, pAIM11, which has a deletion removing the entire region, was positive for luciferase in the first lysate but unable to transfer luciferase gene expression in subsequent passaging attempts, either in the absence or presence of a complementing left end fragment.

A more discrete mutant (pAIM21) disrupts only three of the unknown ORFs but leaves intact the dUTPase and the REP-like ORFs. However, similar to pAIM11, the pAIM21 genome was also unable to transfer luciferase gene expression in subsequent passaging attempts either in the absence or presence of a complementing left end fragment (Figure 2B). Thus both of the mutations alter sequences that must be present *in cis* for virus replication. pAIM27 deletes only REP, while pAIM25 and 26 delete the duTPase, REP and one unknown open reading frame. These three genomes all produced luciferase activity in the first lysate. Subsequent passage of the material revealed that CELO AIM 25, 26 and 27 were not capable of replicating in the absence of complementation. However, unlike pAIM11 and 21, passageable luciferase activity was observed if the initial transfection contained the complementing left end plasmid (Figure 2B). These three complemented viruses (CELO AIM25+, 26+ and 27+) were amplified for an additional 6 passages in LMH cells with, surprisingly, only modest declines in their ability to transduce luciferase activity (results not shown). PCR and Southern analysis revealed a substantial contribution of apparently wildtype CELO in the passage 3 material, demonstrating that recombination had occurred that re-introduced the sequences deleted in the original mutants. Thus, an apparently wildtype CELO was produced and provided complementation functions for the luciferase-bearing mutant CELO.

In conclusion, of the left end sequences, a region was identified (between 2981 and 4334) that is essential *in cis* for virus replication. A second region was identified (between nt 938 and 2900) which is essential for virus replication but could be supplied *in trans*. Formally, it is possible that a series of recombination events generated a viral genome that contained both the originally deleted sequence and the luciferase cassette, however, the simplest explanation of this pattern is that a simple recombination occured between pB5.5 and the mutant CELO genome to generate a wildtype CELO genome, which in subsequent passages provided complementation activity for a small number of the mutant (luciferase positive) viruses in the mixture. Some of these viral genomes contain net insertions of sequence over the wildtype size with the largest containing a 1616 bp deletion combined with a 3.3 kb luciferase cassette insertion. Thus CELO, which in the wildtype form is already 8 kb larger than Ad5, can package, at least, an additional 1700 bp of sequence.

### Example 3

A portion of the CELO right end is dispensable

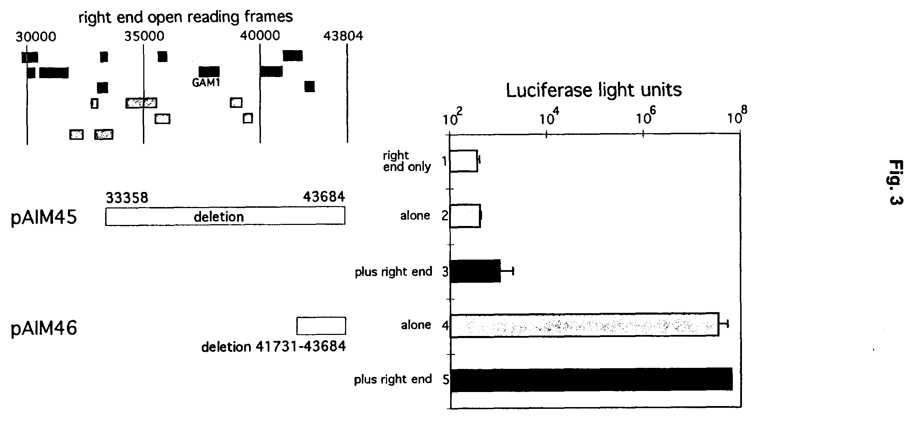

A similar mutational strategy was used for an initial analysis of the right end sequences of CELO. The genome plasmid pAIM45 contains a large deletion from 33358-43684, deleting 10 ORFs of 99 amino acids or larger, including the previously characterized GAM1 gene (Figure 3, ref. 7). The plasmid pAIM46 contains a more discrete deletion from 41731-43684 and disrupts two ORFs (Figure 3 depicts the analysis of CELO right end mutations. Analysis was performed as outlined in Example 3, Figure 2B except that the right end complementing plasmid pB13.3 was used in place of the pB5.5 (lanes labeled "plus right end). Both mutant genomes included a luciferase cassette in place of the deleted sequences.

pAIM45 and pAIM46 were transfected into LMH cells either alone or with a plasmid bearing the wildtype CELO right end sequences (pB13.3). Luciferase activity was obtained in lysates of the transfected cells demonstrating successful transfection (results not shown). Subsequent passage of the material on fresh LMH cells revealed that pAIM45, with the extensive right end deletion, was not capable of generating infectious CELO particles, neither in the absence nor in the presence of the intact right end sequences (Figure 3). Not surprisingly, this extensive deletion removed sequences that were essential, and most likely some of these are required *in cis*, as evidenced by the absence of complementation by the wildtype right end sequences. In contrast, pAIM46 genome was found to generate infectious and passageable virus in both the presence or the absence of the complementing genome fragment (Figure 3). The two disrupted open reading frames in pAIM46 are thus dispensable for cell culture growth of CELO as well as for growth in chicken embryos (see Example 5).

To verify the structure of pAIM46 and of the genome carried by CELO AIM46, a PCR analysis was performed to demonstrate that the deletion/insertion constructed in the plasmid was maintained in the genome of the amplified CELO AIM46 virus.

The primers used for PCR are the following:
OAIM 24: (CCGAGAATCCACCAATCGTA) is a sense oligo hybridizing in CELO right end (at nt 41699).
OAIM25: (CAGCGTGTCGCTATACGCAA) is an antisense oligo hybridizing in the CELO right end (at nt 43752).
OAIM26: (GCGATGACGAAATTCTTAGC) is a sense oligo hybridizing in the luciferase expression cassette.

PCR with OAIM24 and 25 should give a 2053 bp product with a wildtype CELO template and a 3422 bp product with the AIM46 template. PCR with OAIM24 and 26 should give a 958 bp product with AIM46 template and no product with wildtype CELO template.

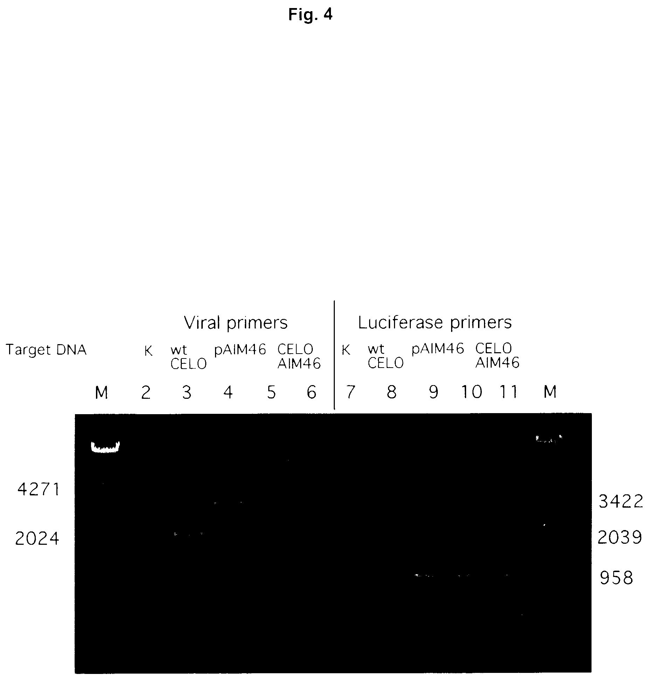

The result of the PCR analysis of wt CELO vs. CELO AIM46 is shown in Figure 4:

Lanes marked M: marker DNA, (EcoR1/HindIII cut Lambda DNA); lanes 2 to 6: primers OAIM24+OAIM26 were used with, lane 2: irrelevant target DNA; lane 3: wt CELO DNA; lane 4: plasmid pAIM46 DNA; lane 5 and 6: DNA isolated from CELO AIM46; Lanes 7 to 11: primers OAIM24 and OAIM26were used with, lane 7: irrelevant DNA; lane 8: wt CELO DNA; lane 9: plasmid pAIM46 DNA; lanes 10 and 11: DNA isolated from CELO AIM46 DNA. DNA sizes (in base pair) are indicated for some of the marker molecules (left side) and for the expected PCR products (right side).

As shown in Figure 4, both the plasmid pAIM46 and DNA isolated from the virus CELO AIM46 produced the expected PCR products. Primers that span the deletion/insertion site generate the predicted PCR product of 3422 bp with pAIM46 target DNA (Figure 4, lane 4) and with DNA derived from two CELO AIM46 preparations (Figure 4, lanes 5 and 6)while PCR with wildtype CELO DNA produces the predicted DNA molecule of 2039 bp (Figure 4, lane 3). Furthermore, primers that recognize the luciferase insert produce the predicted 958 bp product from DNA derived from pAIM46 or from two isolates of CELO AIM46 (Figure 4, lanes 9-11), but not from DNA derived from wildtype CELO (Figure 4, lane 8).

### Example 4

### Immunofluorescence analysis of CELO AIM46 vs. wildtype CELO replication

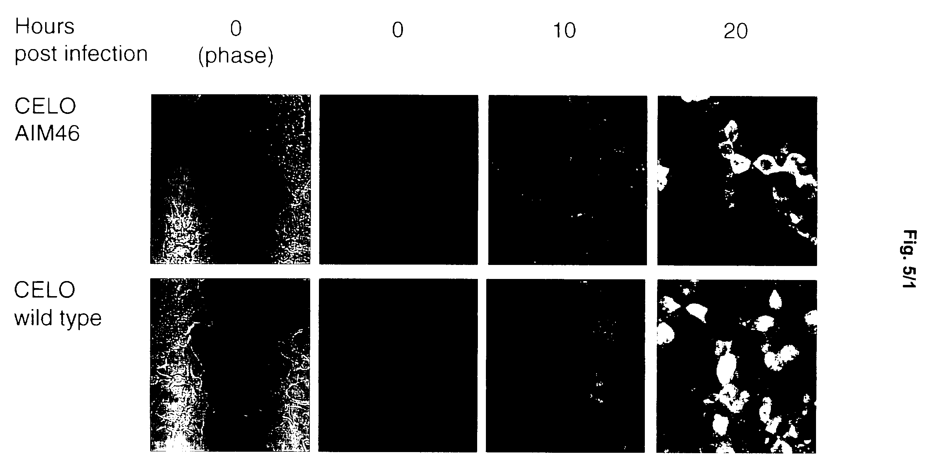

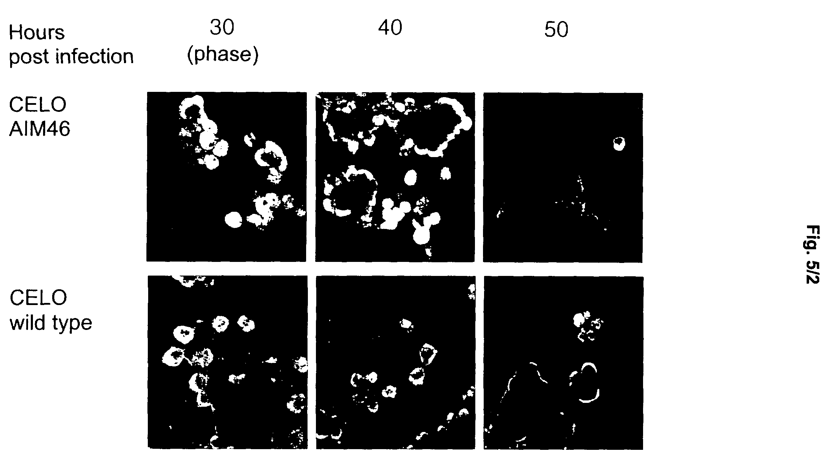

Luciferase data showed that CELO AIM46 can replicate in LMH cells in the absence of complementation. To analyze more directly the replication of CELO AIM46 in comparison to wildtype CELO, the two virus types were used to infect LMH cells and the progression of virus infection was monitored by immunofluorescence microscopy using a polyclonal antiserum directed against CELO capsid proteins. Figure 5 shows the immunofluorescence, monitoring the replication of wildtype CELO vs. CELO AIM46. LMH cultures were infected at 500 particles per cell with either CELO AIM46 (upper row) or wildtype CELO (lower row). Cell samples were fixed at the indicated times post-infection and production of CELO virion proteins was monitored by immunofluorescence using an antiserum againt CELO virion. For both wildtype CELO and CELO AIM46, replication is first detectable at 10 hours post-infection and the signal increases over the next 30 hours until cytopathic effect results in detachment of cells and a decline in the fluorescence signal. Thus, in a cell culture infection, CELO AIM 46 appears to replicate with kinetics that are similar to wildtype CELO.

### Example 5

### Growth of CELO AIM46 in chicken embryos

In the initial stages of this work, LMH cells were used for cell culture propagation of CELO AIM46. Because the nature of the transformation event that established this cell line is not clear, it remains possible that the LMH cells provide some helper functions for CELO AIM46 that wildtype chicken embryonic cells might lack. This experiment should also determine if CELO AIM46 was capable of growing in chicken embryos for practical considerations: the low cost and ease of handling of embryos would facilitate production of these viruses. Equal quantities of either wildtype CELO or CELO AIM46 were injected into the allantoic cavity of 9 day old chicken embryos. After incubation at 37 °C for 4 days, the allantoic fluid was harvested and virus was purified by banding in CsCl density gradients. Yields of purified wildtype CELO ranged from 0.149 to 0.9 mg per egg (average: 0.427) mg/egg) while CELO AIM46 yields were from 0.119 to 0.828 mg per egg (average: 0.301 mg/egg; Table 3). The modifications introduced in CELO AIM46 appear to effect the growth of AIM46 in chicken embryos to only a modest extent.

### Example 6

### Physical stability of CELO AIM46

A distinctive feature of the CELO virion is physical stability, most readily measured by resistance of the virion to elevated temperatures. While mastadenoviruses such as Ad5 are inactivated by exposure to temperatures of 48°C and higher (4, 8, 16), CELO was originally reported to be stable at 56°C (57) and subsequent isolates of the virus have been reported with stability at higher temperatures as well as to other harsh treatments (reviewed in 39). The molecular nature of the CELO stability has not been determined. A major component of the Ad5 capsid stability, pIX, has not been identified in CELO. Perhaps hexon or other capsid components have altered sequences which allow more stable protein/protein interactions. It is likely that this stability is important in the wild for CELO virus survival in the harsh avian environment. In any case, it was of interest to determine if the CELO recombinant vector retains the stability of the wildtype CELO virion.

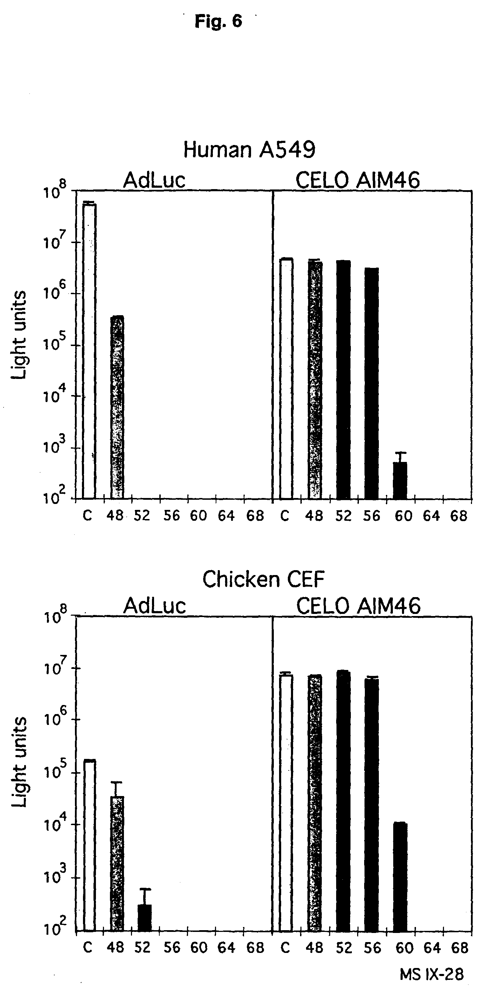

A recombinant adenovirus type 5 bearing a luciferase expression unit (AdLuc) and CELO AIM45 were exposed to a heat titration (30 minute exposure to defined temperatures from 42 to 68°C). Subsequently, each sample was tested for its ability to transfer luciferase activity to either human A549 or avian CEF cells. Figure 6 shows the immunofluorescence assays monitoring the replication of wildtype CELO vs. CELO AIM46. LMH cultures were infected at 500 particles per cell with either CELO AIM46 (upper row) or wildtype CELO (lower row). Cell samples were fixed at the indicated times post-infection and production of CELO virion proteins was monitored by immunofluorescence using an antiserum againt CELO virion.

As previously demonstrated for Ad5, the virus capsid, and thus the transduction ability of the virus is sensitive to heat (4, 3, 16). Ad5 transduction of human cells declines by a factor of more than 100 when exposed to 43°C for 30 minutes and is inactivated at 52°C and higher temperatures (Figure 6). In strong contrast, CELO AIM46 transduction ability is not affected by heating at 56°C and the virus only begins to lose activity when exposed to 60°C for 30 minutes (Figure 6). It was found that transduction with wildtype CELO displays similar heat stability indicating that the alterations introduced in CELO AIM46 do not significantly alter the virion's stability.

### Example 7

### CELO can transduce a variety of cell types

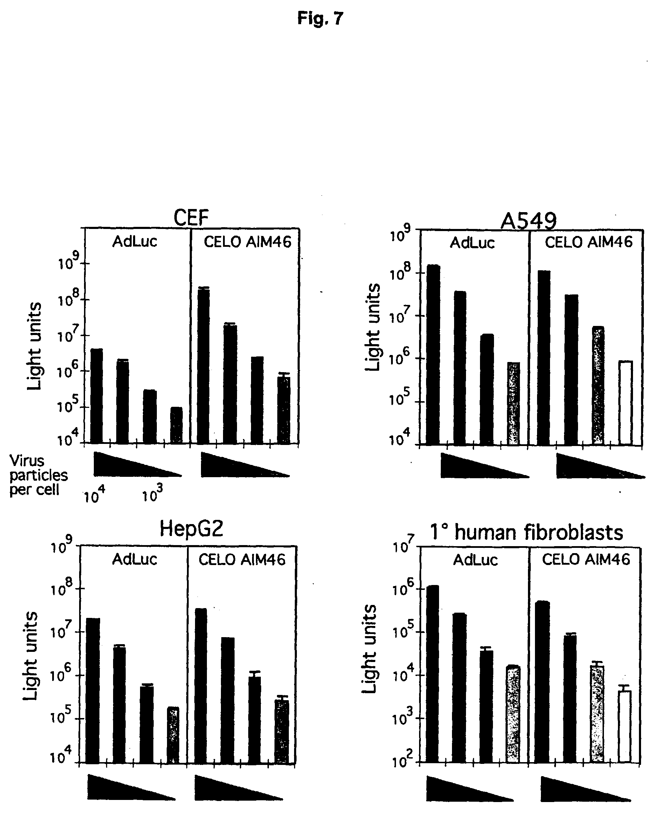

In considering future applications, it is of interest to determine the types of cells that can be transduced by a CELO-based vector. A panel of commonly used mammalian and chicken cell types was tested for their transducibility by CELO AIM46. For comparison, the Ad5 derivative Adluc carrying the same luciferase expression cassette was used. The results for four of these cell types are presented in Figure 7 which shows the tropism of CELO vs. Ad5. The indicated cell types were exposed to a AdLuc or CELO AIM46 at 10000, 3000, 1000, or 300 particles per cell (see methods section for the protocol for 24 well plate). At 24 hours post-infection, luciferase activity was determined. The values are the average of three transductions with the standard deviations indicated Cells of avian origin (e.g. the chicken fibroblast line CEF38) were transduced with nearly 100-fold greater efficiency with the CELO vector than with the human AdLuc (Figure 7). Note that CEF38 cells do not support virus replication, so the difference between the Ad5 vector and the CELO vector cannot be ascribed to an amplification associated with virus replication and must be due to primary transduction or gene expression effects. In the human cell types tested, CELO worked comparable the Ad5 vector. These include the hepatoma line HepG2, the lung epithelial carcinoma line A549 and primary human dermal fibroblast (Figure 7). Similar results were obtained with the human carcinoma line HeLa, the murine myoblast line C2C12, and the canine epithelial line MDCK.

In conclusion, it was found that CELO AIM46 is capable of transducing avian cells approximately 100-fold more efficiently than a human Ad5 vector. Surprisingly, CELO AIM46 is also transduces mammalian cell types with efficiencies comparable to an Ad5 based vector.

### Example 8

### GFP expression from adenovirus and CELO vectors.

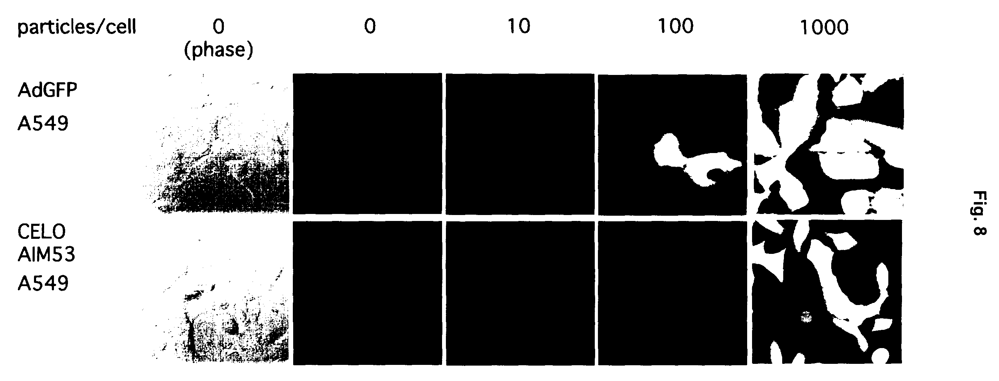

The green fluorescent protein (GFP) has emerged as a useful marker for gene transfer studies. Accordingly, a CELO vector (CELO AIM53) expressing EGFP (Clontech) in the CELO AIM46 background was prepared. This vector was compared to an Ad5 vector bearing the same CMV/EGFP/βglobin expression unit. It was found that both vectors function to transfer a GFP gene in human A549 cells. The results are shown in Figure 8, which depicts the transduction of EGFP using recombinant Ad5 or CELO vectors. The EGFP expressing adenovirus AdGFP and CELO AIM53 were used to infect human A549 cells over a range of virus/cell ratios (10 to 1000 particles per cell). At 24 hours post-infection, cells were fixed and GFP expression was monitored by fluorescence microscopy. Although immunofluorescence with GFP is not quantitative in this format, it appears that, similar to the luciferase recombinants, there are not large differences in transduction capacity between the CELO and the Ad5 EGFP viruses when transducing human A549 cells.

### Example 9

### Further deletions of the CELO right end

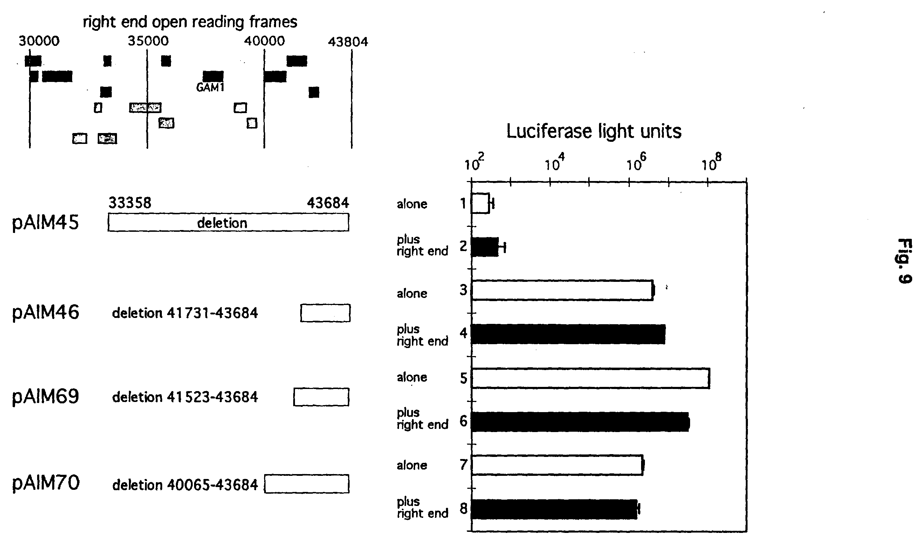

A similar mutational strategy as in Example 3 was used to generate additional deletions in the right end sequences of CELO. The genome plasmid pAIM69 contains a deletion from 41523-43684, disrupting the same two open reading frames that are affected in CELO AIM46. The genome plasmid pAIM70 contains a slightly larger deletion from 40065-43684 and disrupts the same two ORFs as AIM46 and AIM 69 plus one additional ORF. Analysis was performed as outlined in Example 3, Figure 2B, except that the right end complementing plasmid pB13.3 was used in place of the pB5.5 (Fig. 9, lanes labeled "plus right end"). Both mutant genomes included a luciferase cassette in place of the deleted sequences.

pAIM69 and pAIM70 were transfected into LMH cells either alone or with a plasmid bearing the wildtype CELO right end sequences (pB13.3). Luciferase activity was obtained in lysates of the transfected cells demonstrating successful transfection. Subsequent passage of the material on fresh LMH cells revealed that both AIM69 and AIM70 were capable of generating infectious CELO particles in the absence of the intact right end sequences (Figure 9). As shown for CELO AIM46, the two disrupted open reading frames in pAIM69 are thus dispensable for cell culture growth of CELO. The additional open reading disrupted in AIM70 is apparently also dispensable for cell culture growth.

**Table 1**

| **Plasmids used in CELO recombinant construction.** | | | |
|---|---|---|---|
| **Plasmid name** | **CELO left end sequences** | **CELO right end sequences** | **Comments** |
| pWü-H35 | 1-1601 | 42845-43804 | Left and right terminal CELO HindIII fragments cloned with flanking SpeI linkers in pBR327 |
| pB5.5 | 1-5503 | -- | Left terminal CELO HpaI fragment in pBluescript |
| pB13.3 | -- | 30502-43804 | Right terminal CELO HpaI fragment in pBluescript |
| pWüHpa | 1-5503 | 30502-43804 | Left and right terminal CELO HpaI fragments, with flanking SpeI linkers in pBR327 |
| pAIM3 | 1-5503 | 30502-30643 | pWüHpa derivative (removal of Asp718 fragment) |
| | | 40064-43804 | |
| pAIM7 (luc*) | 1-1069 | 30502-30643 | pWüHpa derivative transfer vector for pAIM11 |
| | 4339-5503 | 40064-43804 | |
| pAIM16 (luc) | 1-2981 | 30502-30643 | pWüHpa derivative transfer vector for pAIM21 |
| | 4339-5503 | 40064-43804 | |
| pAIM22 (luc) | 1-940 | 30502-30643 | pWüHpa derivative transfer vector for pAIM25 |
| | 2303-5503 | 40064-43804 | |
| AIM23 (luc) | 1-1069 | 30502-30643 | pWüHpa derivative transfer vector for pAIM26 |
| | 2681-5503 | 40064-43804 | |
| pAIM24 (luc) | 1-1689 | 30502-30643 | pWüHpa derivative transfer vector for pAIM27 |
| | 2903-5503 | 40064-43804 | |
| pAIM43 (luc) | 1-5503 | 30502-30643 | pWüHpa derivative transfer vector for pAIM45 |
| | | 40064-43804 | |
| pAIM44 (luc) | 1-5503 | 30502-30643 | pWüHpa derivative transfer vector for pAIM46 |
| | | 40064-43804 | |
| pAIM52 (EGFP) | -- | -- | transfer vector for homologous recombination with pAIM44 |

| | | | |
|---|---|---|---|
| luc* = luciferase | | | |

**Table 2**

| **Plasmids containing CELO variants** | | |
|---|---|---|
| **CELO genome construct** | **Celo sequences deleted** | **Replication in LMH cells** |
| | | |

| **CELO Left end** | | |
|---|---|---|
| pAIM11 | 1065-4334 (3269 bp) AatII+NcoI | Defective, cannot be complemented |
| pAIM25 | 938-2300 (1362 bp) Eco47-3 | Defective, can be complemented |
| pAIM26 | 1065-2681 (1616 bp) AatII+SphI | Defective, can be complemented |
| pAIM27 | 1687-2900 (1213 bp) PmaCI | Defective, can be complemented |
| pAIM21 | 2981-4334 (1353 bp) StyI | Defective, cannot be complemented |

| **CELO Right end** | | |
|---|---|---|
| pAIM45 | 33358-43684 (10326 bp) EcoRV | Defective, cannot be complemented |
| pAIM46 | 41731-43684 (1953 bp) EcoRV | Replication competent |
| pAIM53 | | |
| pAIM69 | 41523-43684 (2161 bp) PvuII-EcoRV | Replication competent |
| pAIM70 | 40065-43684 (3619) Asp718+EcoRV | Replication competent |

**Table 3**

| **Yield of CELO virus from eggs.**^{**1**} | | | | | |
|---|---|---|---|---|---|
| Virus type | Preparation # | yield (mg CsCl purified virus)² | number of eggs | virus per egg (mg) | average yield per egg (mg) |
| wildtype CELO | 1 | 1.80 | 2 | 0.90 | 0.427 |
| wildtype CELO | 2 | 0.496 | 2 | 0.248 | |
| wildtype CELO | 3 | 0.345 | 2 | 0.173 | |
| wildtype CELO | 4 | 1.33 | 2 | 0.665 | |
| wildtype CELO | 5 | 5.96 | 40 | 0.149 | |
| CELO AIM 46 | 1 | 1.66 | 2 | 0.828 | 0.301 |
| CELO AIM 46 | 2 | 0.133 | 1 | 0.133 | |
| CELO AIM 46 | 3 | 0.247 | 1 | 0.247 | |
| CELO AIM 46 | 4 | 0.618 | 2 | 0.309 | |
| CELO AIM 46 | 5 | 0.340 | 2 | 0.170 | |
| CELO AIM 46 | 6 | 0.237 | 2 | 0.119 | |

| | | | | | |
|---|---|---|---|---|---|
| Notes 1. Wildtype CELO or CELO AIM46 (8 x 10⁸ particles in 100 µl of HBS) were injected into the allantoic cavity of 9 day old chicken embryos. After 4 days of incubation at 370°C, allantoic fluid was harvested and virus was purified by banding in CsCl density gradients as previously described (9). | | | | | |
| 2. The virus yield is expressed as purified virus protein. Protein was measured by Bradford assay using bovine serum albumin as a standard. | | | | | |

### REFERENCES

1. Baker, A., M. Saltik, H. Lehrmann, I. Killisch, V. Mautner, G. Lamm, G. Christofori, and M. Cotten. 1997. Polyethylenimine is a simple, inexpensive and effective reagent for condensing and linking plasmid DNA to adenovirus for gene delivery. Gene Therapy 4: 773-782.
2. Bett A.J., W. Haddara, L. Prevec, F.L.Graham. 1994. An efficient and flexible system for construction of adenovirus vectors with insertions or deletions in early regions 1 and 3. Proc Natl Acad Sci USA 91:8802-8806
3. Boussif O., F. Lezoualc'h, M.A. Zanta, M.D. Mergny, D. Scherman, B. Demeneix, and J.-P.Behr. 1995. A versatile vector for gene and oligonucleotide transfer into cells in culture and in vivo: Polyethylenimine. Proc. Natl. Acad. Sci. U S A. 92: 7297-7301.
4. Caravokyri, C. and K.N. Leppard. 1995. Constitutive episomal expression of polypeptide IX (pIX) in a 293-based cell line complements the deficiency of pIX mutant adenovirus type 5. J. Virol. 69: 6627-6633.
5. Chartier C., B. Degryse, M. Gantzer, A. Dieterle, A. Pavirani, and N. Mehtali. 1996. Efficient generation of recombinant adenovirus vectors by homologous recombination in Escherichia coli. J. Virol. 70: 4805-4810.
6. Chiocca, S., R. Kurzbauer, G.Schaffner, A. Baker, V. Mautner, and M. Cotten. 1996. The complete DNA sequence and genomic organization of the avian adenovirus CELO. J. Virol. 70: 2939-2949.
7. Chiocca, S., A. Baker, and M. Cotten. 1997. Identification of a novel anti-apoptotic protein, GAM-1, encoded by the CELO adenovirus. J. Virol. 71: 3168-3177.
8. Colby, W.W. and T.Shenk. 1981. Adenovirus type 5 virions can be assembled in vivo in the absence of detectable polypeptide IX. J. Virol. 39: 977-980.
9. Cotten, M., E. Wagner, K.Zatloukal, and M.L. Birnstiel. 1993. Chicken adenovirus (CELO virus) particles augment receptor-mediated DNA delivery to mammalian cells and yield exceptional levels of stable transformants, J. Virol. 67:3777-3785.
10. Cowen, B., B.W. Calnek, N.A. Menendez and R.F. Ball. 1978. Avian adenoviruses: effect on egg production, shell quality and feed consumption. Avian Diseases 22: 459-470.
11. Crouzet J., L. Naudin, C. Orsini, E. Vigne, L. Ferrero, A. Le Roux, P. Benoit, M. Latta, C, Torrent, D. Branellec, P. Denefle, J.F. Mayaux, M. Perricaudet, and P. Yeh 1997. Recombinational construction in Escherichia coli of infectious adenoviral genomes.Proc Natl Acad Sci USA 94: 1414-1419.
12. DeGregori J., G. Leone, A. Miron, L. Jakoi, and J.R. Nevins. 1997. Distinct roles for E2F proteins in cell growth control and apoptosis. Proc Natl Acad Sci USA 94:7245-7250.
13. Degryse, E. 1996. In vivo intermolecular recombination in Escherichia coli: applications to plasmid construction. Gene 170: 45-50.
14. de Wet J.R., K.V. Wood, M. DeLuca , D.R. Helinski, and S. Subramani. 1987. Firefly luciferase gene: structure and expression in mammalian cells. Mol Cell Biol 7:725-737.
15. Fisher K.J., H. Choi, J. Burda, S.J. Chen, and J.M. Wilson 1996. Recombinant adenovirus deleted of all viral genes for gene therapy of cystic fibrosis. Virology 217: 11-22.
16. Ghosh-Choudhury, G., Y. Haj-Ahmad, and F.L. Graham. 1987. Protein IX, a minor component of the human adenovirus capsid, is essential for the packaging of full length genomes. EMBO J. 6: 1733-1739.
17. Gillen, J.R., D.K.Willis, and A.J.Clark. 1974. in Mechanisms in Genetic Recombination (R.F. Greil, ed.) Plenum, New York, pp123-135.
18. Gluzman, Y., H. Reichl, and D. Solnick, 1982. Helper-free adenovirus type 5 vectors. p187-192. in Y. Gluzman (ed.) Eukaryotic Viral Vectors. Cold Spring Harbor Laboratory Press, Cold Spring Harbor, NY.
19. Graham F.L., J. Smiley, W.C. Russell, and R. Nairn. 1977. Characteristics of a human cell line transformed by DNA from human adenovirus type 5. J. Gen. Virol. 36:59-74.
20. Grubb B.R., R.J. Pickles, H. Ye, J.R. Yankaskas, R.N.Vick, J.F. Engelhardt, J.M. Wilson, L.G.Johnson, and R.C. Boucher 1994. Inefficient gene transfer by adenovirus vector to cystic fibrosis airway epithelia of mice and humans. Nature 371: 802-806.
21. Hardy S., M. Kitamura, T. Harris-Stansil, Y. Dai, M.L.Phipps. 1997. Construction of adenovirus vectors through Cre-lox recombination. J Virol 71: 1842-1849.
22. Hay, R.T., N.D. Stow, and I.M. McDougall. 1984. Replication of adenovirus mini-chromosomes. J. Mol. Biol. 175: 493-510.
23. He T.C., S. Zhou, L.T. da Costa, J. Yu, K.W. Kinzler, and B.A. Vogelstein. 1998. simplified system for generating recombinant adenoviruses. Proc Natl Acad Sci USA 95:2509-2514.
24. Hess, M., A. Cuzange, R.W.H. Ruigrok, J. Chroboczek and B. Jacrot. 1995. The avian adenovirus penton: two fibres and one base. J. Mol. Biol. 252:379-385.
25. Horwitz, M.S. 1996. Adenoviruses. pp2149-2171 in Fields Virology, Third Edition, edited by B.N. Fields, D.M. Knipe, P.M. Howley et al., Lippincott-Raven Publishers, Philidelphia.
26. Imler J.L., C. Chartier, A. Dieterle, D. Dreyer, M. Mehtali, and A. Pavirani. 1995. An efficient procedure to select and recover recombinant adenovirus vectors. Gene Ther. 2: 263-268.
27. Kawaguchi T., K. Nomura, Y. Hirayama, T. Kitagawa. 1987. Establishment and characterization of a chicken hepatocellular carcinoma cell line, LMH. Cancer Res. 47: 4460-4464.
28. Karlsson S., R.K. Humphries, Y.Gluzman, and A.W.Nienhuis. 1985. Transfer of genes into hematopoletic cells using recombinant DNA viruses. Proc Natl Acad Sci USA 82: 158-162.
29. Khatri A., Z.Z. Xu, and G.W. Both. 1997. Gene expression by atypical recombinant ovine adenovirus vectors during abortive infection of human and animal cells in vitro. Virology 239: 226-237.
30. Klonjkowski B., P. Gilardi-Hebenstreit, J. Hadchouel, V. Randrianarison, S. Boutin, P. Yeh , N. Perricaudet, and E.J. Kremer. 1997. A recombinant E1-deleted canine adenoviral vector capable of transduction and expression of a transgene in human-derived cells and in vivo. Hum. Gene Ther. 8: 2103-2115
31. Kovesdi I., D.E. Brough, J.T. Bruder, and T.J. Wickham. 1997. Adenoviral vectors for gene transfer. Curr. Opin. Biotechnol. 8: 583-589
32. Kumar-Singh R., and J.S. Chamberlain. 1996. Encapsidated adenovirus minichromosomes allow delivery and expression of a 14 kb dystrophin cDNA to muscle cells. Hum Mol Genet 5: 913-921.
33. Laver, W.G., H.B. Younghusband and N.G. Wrigley. 1971. Purification and properties of chick embryo lethal orphan virus (an avian adenovirus). Virology 45: 598-614.
34. Lemay P., M.L. Boudin, N. Milleville, and P. Boulanger. 1980. Human adenovirus type 2 protein IIIa. I. Purification and characterization. Virology 101: 131-143.
35. Li, P., A.J.D. Bellett and C.R. Parish. 1984a. The structural proteins of Chick Embryo Lethal Orphan Virus (Fowl Adenovirus Type I). J. Gen. Virol. 65: 1803-1815.
36. Li, P., A.J.D. Bellett and C.R. Parish. 1984b. Structural organization and polypeptide composition of the avian adenovirus core. J. Virol. 52: 638-649.
37. Li, P., A.J.D. Bellett and C.R. Parish. 1984c. DNA-binding proteins of chick embryo lethal orphan virus: lack of complementation between early proteins of avian and human adenoviruses. J. Virol. 65: 1817-1825.
38. Lieber A., C.Y. He, I. Kirillova, and M.A. Kay. 1996. Recombinant adenoviruses with large deletions generated by Cre-mediated excision exhibit different biological properties compared with first-generation vectors in vitro and in vivo. J Virol 70: 8944-8960.
39. McFerran, J.B. and B.M. Adair. 1977. Avian adenoviruses - a review. Avian Pathology 6: 189-217.
40. Mittal S.K., L. Prevec, F.L. Graham, and L.A. Babiuk. 1995. Development of a bovine adenovirus type 3-based expression vector. J. Gen. Virol. 76:93-102.
41. Miyake S., M. Makimura, Y. Kanegae, S. Harada, Y. Sato, K. Takamori, C. Tokuda, and I. Saito. 1996. Efficient generation of recombinant adenoviruses using adenovirus DNA-terminal protein complex and a cosmid bearing the full-length virus genome. Proc Natl Acad Sci USA 93: 1320-1324.
42. Oliner, J. D., K.W. Kinzler, and B. Vogelstein. 1993. In vivo cloning of PCR products in E. coli. Nucleic Acids Research 21: 5192-5197.
43. Parks R.J., L. Chen, M. Anton, U. Sankar, M.A. Rudnicki, F.L.Graham. 1996. A helper-dependent adenovirus vector system: removal of helper virus by Cre-mediated excision of the viral packaging signal. Proc Natl Acad Sci USA 93: 13565-13570.
44. Petersson, U. and R.J. Roberts. 1986. Adenovirus gene expression and replication: a historical review, in DNA Tumor Viruses: Control of Gene Expression and Replication pp 37-57.Cold Spring Harbor Laboratory Press, Cold Spring Harbor, New York.
45. Plank C., K. Zatloukal, M. Cotten, K. Mechtler, and E. Wagner. 1992. Gene transfer into hepatocytes using asialoglycoprotein receptor mediated endocytosis of DNA complexed with an artificial tetra-antennary galactose ligand. Bioconjugate Chemistry. 3: 533-539.
46. Polyak K., Y. Xia, J.L. Zweier, K.W. Kinzler, and B. Vogelstein. 1997. model for p53-induced apoptosis. Nature 389: 300-305.
47. Robinson A.J., H.B.Younghusband, and A.J. Bellett. 1973. A circular DNA-protein complex from adenoviruses. Virology 56:54-69.
48. Schaack J., S. Langer, X. Guo. 1995. Efficient selection of recombinant adenoviruses by vectors that express beta-galactosidase.J Virol. 69: 3920-3923.
49. Shenk, T. 1995. Group C adenovirus as vectors for gene therapy.pp 2111-2148 in Viral Vectors (eds. M.G. Kaplitt and A.D. Loewy) pp. 43-54. Academic Press, San Diego.
50. Shenk, T. 1996. Adenoviridae: The viruses and their replication, in Fields Virology, Third Edition. edited by B.N. Fields, D.M. Knipe, P.M. Howley et al., Lippincott-Raven Publishers, Philidelphia.
51. Soberon X., L. Covarrubias, F. Bolivar. 1980. Construction and characterization of new cloning vehicles. IV. Deletion derivatives of pBR322 and pBR325. Gene 9: 287-305.
52. Van Doren K., D. Hanahan, and Y. Gluzman. 1984. Infection of eucaryotic cells by helper-independent recombinant adenoviruses: early region 1 is not obligatory for integration of viral DNA. J Virol . 50: 606-614.
53. Vrati S., E.S. Macavoy, Z.Z. Xu, C. Smole, D.B. Boyle, and G.W.Both. 1996b Construction and transfection of ovine adenovirus genomic clones to rescue modified viruses. Virology 220: 200-203.
54. Weiss R.S., S.S. Lee, B.V. Prasad, and R.T. Javier. 1997. Human adenovirus early region 4 open reading frame 1 genes encode growth-transforming proteins that may be distantly related to dUTP pyrophosphatase enzymes. J. Virol. 71:1857-1870.
55. Wilson C., and M.A. Kay. 1995. Immunomodulation to enhance gene therapy. Nature Medicine. 1: 887-889.
56. Xu Z.Z., A. Hyatt, D.B. Boyle and G.W. Both. 1997. Construction of ovine adenovirus recombinants by gene insertion or deletion of related terminal region sequences. Virology 230: 62-71.
57. Yates, V.J. and D.E. Fry. 1957. Observations on a chicken embryo lethal orphan (CELO) virus. Am. J. Vet. Res. 18: 657-660.
58. Zabner J., B.G. Zeiher, E. Friedman, and Welsh M.J. 1996. Adenovirus-mediated gene transfer to ciliated airway epithelia requires prolonged incubation time. J.Virol. 70: 6994-7003.
59. Zabner J., P. Freimuth, A. Puga, A. Fabrega, and Welsh MJ. 1997. Lack of high affinity fiber receptor activity explains the resistance of ciliated airway epithelia to adenovirus infection. J. Clin. Invest. 100: 1144-1149.
60. Zheng B., S.K. Mittal, F.L. Graham, and L. Prevec. 1994. The E1 sequence of bovine adenovirus type 3 and complementation of human adenovirus type 5 E1A function in bovine cells. Virus Res 31: 163-186
61. Lillehoj H.S.,and Trout J.M., 1996, Clin. Microbiol. Rev., Jul. 9(3): 349-360
62. Taylor M.A., and Catchpole J. 1994, Appl. Parasitol, Jun. 35(2): 73-86
63. Shirley M.W., 1992, Br. Vet. J., Nov. 148(6): 479-499
64. Lasher H.N. and Davis V.S., 1997, Avian Dis., Jan. 41(1): 11-19

## Claims

1. Recombinant CELO virus or CELO virus DNA characterized in that the region spanning nucleotides 41731-43684 of the wild type CELO virus genome is deleted.

2. The recombinant CELO virus or CELO virus DNA of claim 1 characterized in that the deletion spans the region nt 41523-43684.

3. The recombinant CELO virus or CELO virus DNA of claim 1 characterized in that the deletion spans the region nt 41002-43684.

4. The recombinant CELO virus or CELO virus DNA of claim 1 characterized in that the deletion spans the region nt 40065-43684.

5. The recombinant CELO virus or CELO virus DNA of any one of claims 1 to 4 characterized in that it contains, in addition to the deletions defined in claims 1 to 4, a deletion spanning nt 794-1330.

6. The recombinant CELO virus DNA of any one of claims 1 to 5 contained on a plasmid that can be replicated in procaryotic or eucaryotic cells.

7. The recombinant CELO-Virus or CELO virus DNA of any one of claims 1 to 6 characterized in that it contains a foreign DNA insertion in place of the deletion(s).

8. The recombinant CELO-Virus or CELO virus DNA of claim 7 characterized in that it contains the foreign DNA insertion in place of the deletion as defined in any one of claims 1 to 4, and/or in place of the deletion defined in claim 5.

9. The recombinant CELO virus or CELO virus DNA of claim 7 or 8 characterized in that the foreign DNA encodes an antigen derived from an animal pathogen.

10. The recombinant CELO virus or CELO virus DNA of claim 9 characterized in that the pathogen is avian.

11. The recombinant CELO virus or CELO virus DNA of claim 7 or 8 characterized in that the foreign DNA encodes a human protein.

12. The recombinant CELO virus or CELO virus DNA of claim 11 characterized in that the DNA encodes a therapeutically active protein.

13. The recombinant CELO virus or CELO virus DNA of claim 12 characterized in the foreign DNA encodes an immunostimulatory protein.

14. The recombinant CELO virus or CELO virus DNA of claim 13 characterized in that the immunostimulatory protein is a cytokine.

15. The recombinant CELO virus or CELO virus DNA of Claim 11 characterized in that the foreign DNA encodes a tumor antigen or a fragment thereof.

16. The recombinant CELO virus or CELO virus DNA of claim 11 characterized in that the foreign DNA encodes an antigen derived from a human pathogen.

17. A method for producing the recombinant CELO virus of any of claims 1 to 16 characterized in that the deletion(s) and optionally insertion(s) are carried out on plasmid-borne CELO virus DNA and that the recombinant CELO virus DNA is introduced into a host that supports virus replication.

18. The method of claim 17 characterized in that the host is a primary cell or a cell of an immortalized cell line.

19. The method of claim 17 characterized in that the host is an avian embryo.

20. The method of claim 17 characterized in that cells defined in claim 18 are transfected with CELO recombinant virus DNA and kept in culture to produce a quantity of virus and that said quantity of virus is injected into and amplified in an avian embryo.

21. The method of claim 20 characterized in that the embryo is chicken.

22. A vaccine against an infectious disease of an animal comprising CELO virus of claim 9.

23. A vaccine against an infectious disease of a bird comprising CELO virus of claim 10.

24. A vaccine against an infectious disease of a human comprising CELO virus of claim 16.

25. A pharmaceutical composition comprising a CELO virus of claim 12.

26. The CELO Virus of any one of claims 13, 14 or 15 for the manufacture of a tumor vaccine.

27. A method for producing a recombinant protein of interest characterized in that the recombinant CELO virus of claim 7, wherein the foreign DNA encodes the protein of interest, is introduced into avian embryos and the virus is allowed to amplify to produce the protein of interest and the protein is recovered.


SEQUENCE LISTING

(1) GENERAL INFORMATION:

(i) APPLICANT:
(A) NAME: Boehringer Ingelheim International GmbH
(B) STREET: Binger Strasse 173
(C) CITY: Ingelheim am Rhein
(E) COUNTRY: Germany
(F) POSTAL CODE (ZIP): 55216
(G) TELEPHONE: 06132/772282
(H) TELEFAX: 06132/774377

(ii) TITLE OF INVENTION: Recombinant CELO virus and
CELO virus DNA

(iii) NUMBER OF SEQUENCES: 3

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO:1:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: synthetic DNA

(xi) SEQUENCE DESCRIPTION: SEQ ID NO:1:

CCGAGAATCC ACCAATCGTA 20

(2) INFORMATION FOR SEQ ID NO:2:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: synthetic DNA

(xi) SEQUENCE DESCRIPTION: SEQ ID NO:2:

CAGCGTGTCG CTATACGCAA 20

(2) INFORMATION FOR SEQ ID NO:3:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: synthetic DNA

(xi) SEQUENCE DESCRIPTION: SEQ ID NO:3:

GCGATGACGA AATTCTTAGC 20